# EUROPEAN PATENT APPLICATION

(11) **EP 3 932 423 A1**
(43) Date of publication of application: **05.01.2022**
(21) Application number: 20763296.9
(22) Date of filing: 26.02.2020
(51) Int. Cl.: A61K 39/145, A61P 31/16, A61P 37/04, A61K 9/70, A61K 47/18, A61K 47/26, A61K 47/36, A61M 37/00

(54) **MICRONEEDLE ARRAY AND METHOD FOR MANUFACTURING MICRONEEDLE ARRAY**

(30) Priority: 27.02.2019 JP 2019034107
(71) Applicant: FUJIFILM Corporation, Tokyo 106-8620 (JP)
(72) Inventor: SAKAI Masaki, Ashigarakami-gun, Kanagawa 258-8577 (JP); KAKITA Kosuke, Ashigarakami-gun, Kanagawa 258-8577 (JP); NAKATSUKASA Akihiro, Ashigarakami-gun, Kanagawa 258-8577 (JP); MORI Hisahiro, Ashigarakami-gun, Kanagawa 258-8577 (JP)
(74) Representative: Klunker IP Patentanwälte PartG mbB
(86) International application number: PCT/JP2020/007559
(87) International publication number: WO 2020/175500

(57) **Abstract**

An object of the present invention is to provide a microneedle array in which the stability of influenza vaccine is satisfactory and the utilization efficiency of the influenza vaccine is high, and a method of producing the same. According to the present invention, provided is a self-dissolving microneedle array including a sheet portion, and a plurality of needle portions which are present on an upper surface of the sheet portion, in which the needle portion contains a water-soluble polymer, influenza vaccine, and meglumine or a salt thereof, and the influenza vaccine is administered into a body by dissolution of the needle portions.

## Description

### Technical Field

The present invention relates to a microneedle array and a method of producing the same. The present invention particularly relates to a microneedle array containing influenza vaccine and a method of producing the same.

### Background Art

In recent years, a dissolution type microneedle array in which a base material formed of a substance that is dissolvable in vivo contains a drug has been developed. Since needles of a microneedle array are thin and short, stimulation to nerves is small. Therefore, a microneedle array is also referred to as a "painless injection".

Patent Document 1 describes a method of producing a transdermal delivery device, including a step (i) of providing a fine projection member having a plurality of fine projections; a step (ii) of providing a biocompatible coating dosage form containing a bioactive drug; a step (iii) of coating the fine projection member with the biocompatible coating dosage form to form a transdermal delivery device; and a step (iv) of packaging the transdermal delivery device under an inert atmosphere condition and/or in a partial vacuum in which the transdermal delivery device has been dried. Patent Document 1 describes that meglumine may be added as a counterion of the biological agent. The microneedle array described in Patent Document 1 is not a self-dissolving microneedle array in which a drug is administered into the body by dissolution of needle portions. In addition, Patent Document 2 describes a drug obtained by stabilizing a protein, which contains meglumine.

### Prior Art Documents

### Patent Documents

Patent Document 1: JP2009-522288A
Patent Document 2: WO08/132363A

### SUMMARY OF THE INVENTION

In the related art, administration of influenza vaccine is performed by subcutaneous and intramuscular injections. However, fear of injection needles, pain during the injection, and mental stress are problems. In order to solve the problems, administration carried out using a microneedle array has been suggested as a method that does not cause pain. In particular, a microneedle array including a vaccine is expected to improve the effectiveness, and an influenza vaccine-including microneedle array is expected to be available.

Since a microneedle array is a fine preparation, the microneedle array is required to have a small volume for containing a drug and contain a drug at a higher concentration than that of an injection. Therefore, the distance between the drugs is decreased, and thus there is a possibility that the aggregation or reaction is likely to occur and the stability of the drug may be impaired. A result that even in a case of the microneedle array including influenza vaccine which has been examined by the present inventors, the stability of the influenza vaccine is impaired as compared with the injection is obtained, and thus there is a demand for improving the stability of the influenza vaccine.

Further, in a case where there is a part where the drug included in a microneedle array is not administered in a living body, a large amount of the drug is wastefully consumed. Therefore, the drug needs to be concentrated on the tip of a needle in the microneedle array.

An object of the present invention is to provide a microneedle array in which the stability of influenza vaccine is satisfactory and the utilization efficiency of the influenza vaccine is high, and a method of producing the same.

As a result of intensive examination conducted by the present inventors in order to achieve the above-described object, it was found that in a microneedle array containing influenza vaccine, the stability of the influenza vaccine with time is improved and tip filling performance of the influenza vaccine is also improved by adding meglumine or a salt thereof to a needle portion containing influenza vaccine. The present invention has been completed based on these findings.

That is, according to the present invention, the following inventions are provided.
(1) A self-dissolving microneedle array comprising: a sheet portion; and a plurality of needle portions which are present on an upper surface of the sheet portion, in which the needle portion contains a water-soluble polymer, influenza vaccine, and meglumine or a salt thereof, and the influenza vaccine is administered into a body by dissolution of the needle portions.
(2) The microneedle array according to (1), in which a content of the water-soluble polymer contained in the needle portion is 10% by mass or greater and 99% by mass or less with respect to a solid content of the needle portion.
(3) The microneedle array according to (1) or (2), in which a content of the meglumine or the salt thereof is in a range of 0.01 times to 300 times a content of the influenza vaccine.
(4) The microneedle array according to any one of (1) to (3), in which the needle portion contains saccharides.
(5) A method of producing the microneedle array according to any one of (1) to (4), the method comprising: a step of concentrating influenza vaccine; a step of forming needle portions using the concentrated influenza vaccine obtained in the above-described step; and a step of forming a sheet portion.
(6) The method of producing the microneedle array according to (5), in which the step of concentrating the influenza vaccine is a step of concentrating the influenza vaccine by centrifugation.

In the influenza vaccine-including microneedle array according to the aspect of the present invention, the stability of the influenza vaccine is satisfactory, and the utilization efficiency of the influenza vaccine is high.

### BRIEF DESCRIPTION OF THE DRAWINGS

Fig. 1 illustrates a region from a needle tip to 600 µm of the microneedle and a region from the needle tip to 800 µm of the microneedle.
Fig. 2A is a perspective view illustrating a conical microneedle, Fig. 2B is a perspective view illustrating a pyramid-like microneedle, and Fig. 2C is a cross-sectional view illustrating a conical and pyramid-like microneedle.
Fig. 3 is a perspective view illustrating a microneedle in another shape.
Fig. 4 is a perspective view illustrating a microneedle in still another shape.
Fig. 5 is a cross-sectional view of the microneedles illustrated in Figs. 3 and 4.
Fig. 6 is a perspective view illustrating a microneedle in another shape.
Fig. 7 is a perspective view illustrating a microneedle in still another shape.
Fig. 8 is a cross-sectional view of the microneedles illustrated in Figs. 6 and 7.
Fig. 9 is a cross-sectional view of a microneedle in another shape in which the inclination (angle) of a side surface of a needle portion is continuously changed.
Figs. 10A to 10C are step views illustrating a method of producing a mold.
Fig. 11 is an enlarged view of a mold.
Fig. 12 is a cross-sectional view illustrating a mold in another shape.
Figs. 13A to 13C are schematic views illustrating a step of filling a mold with an influenza vaccine-containing solution.
Fig. 14 is a perspective view illustrating an end of a nozzle.
Fig. 15 is a partially enlarged view illustrating the end of the nozzle and the mold during the filling.
Fig. 16 is a partially enlarged view illustrating the end of the nozzle and the mold during movement.
Figs. 17A to 17D are views for describing a step of forming another microneedle array.
Figs. 18A to 18C are views for describing a step of forming still another microneedle array.
Fig. 19 is a view for describing a peeling step.
Fig. 20 is a view for describing another peeling step.
Fig. 21 is a view for describing a microneedle array.
Figs. 22A and 22B are respectively a plan view and a side view of an original plate.
Fig. 23 is a schematic view illustrating a filling device used in examples.

### DESCRIPTION OF THE PREFERRED EMBODIMENTS

Hereinafter, embodiments of the present invention will be described in detail.

In the present specification, the expression "containing a drug" means that a drug having an amount enough to exhibit drug efficacy is contained in a case of puncturing the body surface. The expression "not containing a drug" means that a drug having an amount enough to exhibit drug efficacy is not contained, and a range of the amount of the drug covers from a case where the drug is not contained at all to a case where the amount thereof is not enough to exhibit the drug efficacy.

In a microneedle array according to the embodiment of the present invention, a needle portion contains meglumine, and thus the stability of influenza vaccine can be improved and the influenza vaccine can be localized at the tip of the needle portion. The effect that the stability of the influenza vaccine can be improved and the influenza vaccine can be localized at the tip of the needle portion by allowing a needle portion to contain meglumine cannot be expected in the related art. Further, the microneedle array according to the embodiment of the present invention is not intended to use the coating type described in JP2009-522288A. That is, the microneedle array according to the embodiment of the present invention is a self-dissolving microneedle array in which a drug is administered into the body by dissolution of the needle portion.

### [Configuration of microneedle array]

The microneedle array according to the embodiment of the present invention is a self-dissolving microneedle array including a sheet portion, and a plurality of needle portions which are present on an upper surface of the sheet portion, in which each needle portion contains influenza vaccine, a water-soluble polymer, and meglumine or a salt thereof, and the influenza vaccine which is a drug is administered into a body by dissolution of the needle portions.

In the present invention, plural means one or more.

The microneedle array according to the embodiment of the present invention includes at least a sheet portion and needle portions and a drug is carried by the needle portions in order to efficiently administer the drug into the skin.

The microneedle array according to the embodiment of the present invention is a device in which a plurality of needle portions are arranged in an array on the upper surface side of the sheet portion. It is preferable that the needle portions are arranged on the upper surface side of the sheet portion. The needle portions may be arranged directly on the upper surface of the sheet portion or may be arranged on the upper surfaces of frustum portions arranged on the upper surface of the sheet portion.

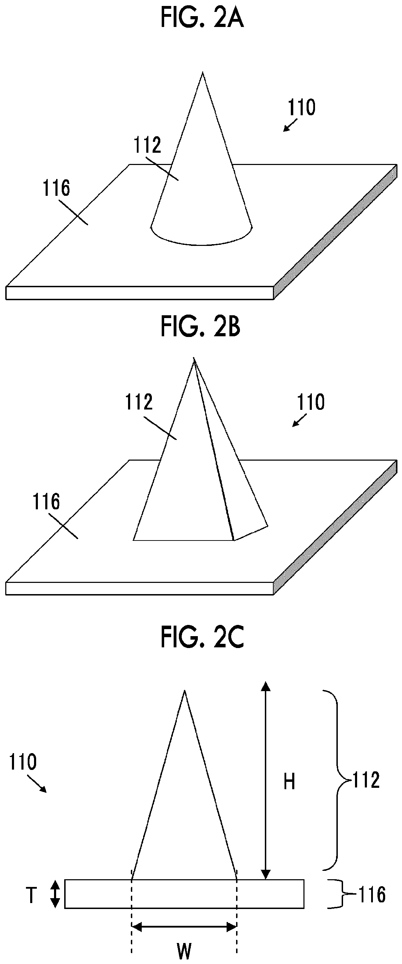

The sheet portion is a foundation for supporting the needle portions and has a planar shape as the shape of a sheet portion 116 illustrated in Figs. 2 to 9. In this case, the upper surface of the sheet portion indicates the surface on which the plurality of needle portions are arranged in an array.

The area of the sheet portion is not particularly limited, but is preferably in a range of 0.005 to 1000 mm², more preferably in a range of 0.1 to 800 mm², and still more preferably in a range of 1 to 800 mm².

The thickness of the sheet portion is a distance between the surface in contact with frustum portions or needle portions and the surface on the opposite side. The thickness of the sheet portion is preferably 1 µm or greater and 2000 µm or less, more preferably 3 µm or greater and 1500 µm or less, and still more preferably 5 µm or greater and 1000 µm or less.

It is preferable that the sheet portion contains a water-soluble polymer. The sheet portion may be formed of a water-soluble polymer or may contain other additives (for example, disaccharides). Further, it is preferable that the sheet portion does not contain a drug.

The water-soluble polymer contained in the sheet portion is not particularly limited, and examples thereof include polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and arabic rubber) and proteins (such as gelatin). The above-described components may be used alone or in the form of a mixture of two or more kinds thereof. Among these, polysaccharides are preferable, hydroxyethyl starch, hydroxypropyl cellulose, hydroxypropyl methyl cellulose, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol are more preferable, and chondroitin sulfate and dextran are particularly preferable.

Disaccharides may be added to the sheet portion and examples of the disaccharides include sucrose, lactulose, lactose, maltose, trehalose, and cellobiose. Among these, sucrose, maltose, and trehalose are particularly preferable.

The microneedle array is formed of a plurality of needle portions arranged in an array on the upper surface side of the sheet portion. The needle portions have a projected structure with a tip, and the shape thereof is not limited to a needle shape having a sharp tip and may be a shape with a blunt tip.

Examples of the shape of a needle portion include a conical shape, a polygonal pyramid shape (square pyramid shape or the like), and a spindle shape. For example, a needle portion may have a shape of the needle portion 112 illustrated in any of Figs. 2A to 9, in which the entire shape of the needle portion may be a conical shape, a polygonal pyramid shape (square pyramid shape or the like), or a shape of a structure in which the inclination (angle) of the side surface of the needle portion is continuously changed. Further, a needle portion may have a multilayer structure with two or more layers, in which the inclination (angle) of the side surface of the needle portion is discontinuously changed.

In a case where the microneedle array according to the embodiment of the present invention is applied to the skin, it is preferable that the needle portions are inserted into the skin and the upper surface or a part of the sheet portion is brought into contact with the skin.

The height (length) of a needle portion indicates the length of a perpendicular line drawn from the tip of the needle portion to the frustum portion or the sheet portion (in a case where a frustum portion is not present). The height (length) of a needle portion is not particularly limited, but is preferably 50 µm or greater and 3000 µm or less, more preferably 100 µm or greater and 1500 µm or less, and still more preferably 100 µm or greater and 1000 µm or less. It is preferable that the length of a needle portion is 50 µm or longer because a drug can be percutaneously administered. Further, it is preferable that the length of a needle portion is 3000 µm or less because occurrence of pain resulting from the contact of needle portions with the nerve is prevented and bleeding can be avoided.

The interface between a frustum portion (or a needle portion in a case where a frustum portion is not present) and the sheet portion is referred to as a base portion. The distance between the farthest points on a base portion of one needle portion is preferably 50 µm or greater and 2000 µm or less, more preferably 100 µm or greater and 1500 µm or less, and still more preferably 100 µm or greater and 1000 µm or less.

The number of needle portions to be arranged in one microneedle array is preferably in a range of 1 to 2000, more preferably in a range of 3 to 1000, and still more preferably in a range of 5 to 500. In a case where one microneedle array includes two needle portions, the interval between needle portions indicates the distance between feet of each perpendicular line drawn from the tip of a needle portion to a frustum portion or the sheet portion (in the case where a frustum portion is not present). In a case where one microneedle array includes three or more needle portions, the interval between needle portions to be arranged indicates an average value obtained by acquiring the distance between a foot of a perpendicular line drawn from the tip of a needle portion to a frustum portion or the sheet portion (in the case where a frustum portion is not present) and a foot of a perpendicular line drawn from the tip of a needle portion nearest to the needle portion to a frustum portion or the sheet portion and averaging the values obtained from all needle portions. The interval between needle portions is preferably 0.1 mm or greater and 10 mm or less, more preferably 0.2 mm or greater and 5 mm or less, and still more preferably 0.3 mm or greater and 3 mm or less.

The needle portions contain influenza vaccine, a water-soluble polymer, and meglumine or a salt thereof. It is preferable that the water-soluble polymer is a biosoluble substance such that a human body is not damaged even in a case where needle portions remain in the skin.

The water-soluble polymer contained in the needle portions is not particularly limited, and examples thereof include polysaccharides (such as hyaluronic acid, sodium hyaluronate, pullulan, dextran, dextrin, sodium chondroitin sulfate, carboxymethyl cellulose, hydroxypropyl cellulose, hydroxyethyl starch, hydroxypropyl methyl cellulose, polyvinylpyrrolidone, polyoxyethylene polyoxypropylene glycol, polyethylene glycol, and arabic rubber) and proteins (such as gelatin). The above-described components may be used alone or in the form of a mixture of two or more kinds thereof. Among these, polysaccharides are preferable, hydroxyethyl starch, hydroxypropyl cellulose, hydroxypropyl methyl cellulose, pullulan, dextran, sodium chondroitin sulfate, sodium hyaluronate, carboxymethyl cellulose, polyvinylpyrrolidone, polyoxyethylene polyoxypropylene glycol, polyethylene glycol, and polyvinyl alcohol are more preferable, and hydroxyethyl starch and dextran are particularly preferable. Further, typically polysaccharides having no electric charge are more preferred because aggregation is unlikely to occur in a case where such polysaccharides are mixed with a drug. The water-soluble polymer contained in the needle portions may be the same as or different from the water-soluble polymer contained in the sheet portion.

The content of the water-soluble polymer contained in the needle portions is preferably 10% by mass or greater and 99% by mass or less, more preferably 10% by mass or greater and 70% by mass or less, and still more preferably 20% by mass or greater and 50% by mass or less with respect to the solid content of the needle portions.

The needle portions contain influenza vaccine as a drug.

The influenza vaccine may contain only one or two or more kinds of virus antigens. In a case where a particular influenza virus is prevalent and a particular strain of vaccine is rapidly produced and supplied, it is preferable that the vaccine contains only one virus antigen. In a case where immunity against a wide range of virus strains is imparted by vaccine administration, it is preferable that the vaccine contains two or more kinds of virus antigens. It is preferable that the influenza vaccine contains an influenza A virus antigen, an influenza B virus antigen, or mixtures thereof. It is more preferable that the influenza vaccine contains an influenza A H1N1 virus antigen, an influenza A H3N2 virus antigen, an influenza B virus antigen, or a mixture thereof. In a case where the influenza vaccine contains two or more kinds of virus antigens, the amount of antigens derived from each virus is not particularly limited, but the influenza vaccine may preferably contain equal amounts of antigens derived from each virus.

The influenza vaccine and the vaccine stock solution may contain a pharmaceutically acceptable carrier as necessary. As the pharmaceutically acceptable carrier, a carrier used to produce a vaccine can be used without limitation, and specifically, saccharides, inorganic salts, buffered saline, dextrose, water, glycerol, isotonic aqueous buffer solutions, surfactants, emulsifiers, preservatives, isotonizing agents, pH adjusters, deactivating agents, and a combination of two or more kinds thereof are appropriately blended.

The influenza vaccine and the vaccine stock solution may contain an immunopotentiator (adjuvant). Examples of the adjuvant include a mineral-containing composition, an oily emulsion, a saponin composition, a virosome, virus-like particles (VLP), and a bacterial or microbial derivative (such as a non-toxic derivative of Enterobacteriaceae lipopolysaccharide, a lipid A derivative, immunostimulatory oligonucleotide ADP ribosylated toxin, or a detoxification derivative thereof).

The content of the influenza vaccine in the entire needle portion is not particularly limited, but is preferably in a range of 0.01 µg to 200 µg in terms of the content of HA per preparation. The content thereof is more preferably in a range of 1 µg to 100 µg. HA is an abbreviation for hemagglutinin.

The mass ratio between the drug and the water-soluble polymer in the needle portion is not particularly limited, but is preferably in a range of 1/0.5 to 1/10 and more preferably in a range of 1/0.5 to 1/4.

In the present invention, the needle portions contain meglumine or a salt thereof. Further, meglumine is also referred to as methyl glucamine (N-methyl-D-glucamine).

Specific examples of the salt of meglumine include a hydrochloride, a sulfate, a carboxylate, a borate, a methanesulfonate, a p-toluenesulfonate, and an ascorbate. Among these, a hydrochloride is preferable.

The content of the meglumine or the salt thereof in the needle portions is preferably in a range of 0.01 times to 300 times the content of the influenza vaccine and more preferably in a range of 0.5 times to 3 times the content of the influenza vaccine.

The needle portions may further contain a soluble additive.

Examples of the soluble additive include saccharides.

As the saccharides, one or more of monosaccharides, disaccharides, oligosaccharides, and polysaccharides can be used. It is preferable that disaccharides can be added to the needle portions. Examples of the disaccharides include sucrose, lactulose, lactose, maltose, trehalose, and cellobiose. Among these, sucrose, maltose, and trehalose are particularly preferable.

In a case where the needle portions contain a soluble additive, the mass ratio between the drug (influenza vaccine) and the soluble additive is not particularly limited, but is preferably in a range of 1/0.1 to 1/10 and more preferably in a range of 1/0.5 to 1/3.

Hereinafter, preferred embodiments of the present invention will be described with reference to the accompanying drawings, but the present invention is not limited thereto.

Figs. 2A to 9 are partially enlarged views illustrating a microneedle 110 of the microneedle array. The microneedle array according to the embodiment of the present invention is configured by formation of a plurality of needle portions 112 on the surface of a sheet portion 116 (in the figures, only one needle portion 112 is shown on the sheet portion 116 or one frustum portion 113 and one needle portion 112 are shown on the sheet portion 116 and this is referred to as the microneedle 110).

The needle portion 112 has a conical shape in Fig. 2A and the needle portion 112 has a square pyramid shape in Fig. 2B. In Fig. 2C, H represents the height of the needle portion 112, W represents the diameter (width) of the needle portion 112, and T represents the height (thickness) of the sheet portion 116.

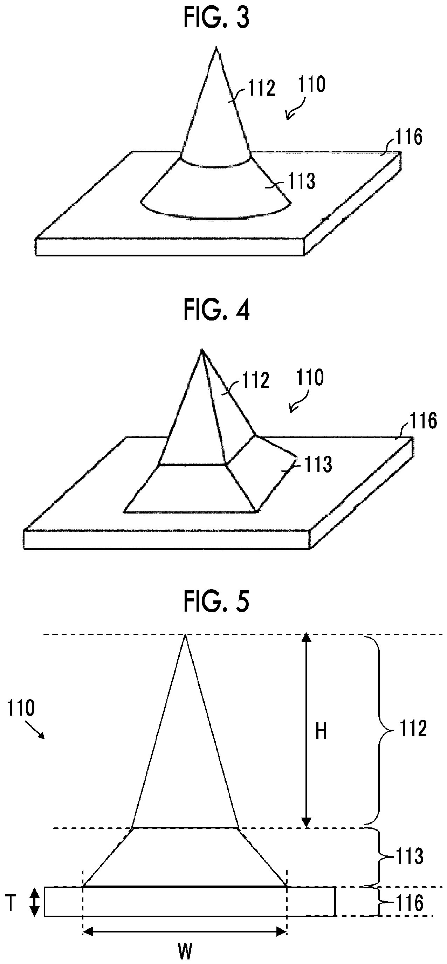

Figs. 3 and 4 illustrate microneedles 110 in different shapes, in which the frustum portion 113 and the needle portion 112 are formed on the surface of the sheet portion 116. In Fig. 3, the frustum portion 113 has a truncated conical shape and the needle portion 112 has a conical shape. In Fig. 4, the frustum portion 113 has a truncated square pyramid shape and the needle portion 112 has a square pyramid shape. However, the shape of the needle portion is not particularly limited.

Fig. 5 is a cross-sectional view illustrating the microneedles 110 illustrated in Figs. 3 and 4. In Fig. 5, H represents the height of the needle portion 112, W represents the diameter (width) of the base portion, and T represents the height (thickness) of the sheet portion 116.

It is preferable that the microneedle array according to the embodiment of the present invention has a shape of the microneedle 110 of Fig. 5 other than the shape of the microneedle 110 in Fig. 2C. With such a configuration, the volume of all needle portions increases so that a greater amount of drug can be concentrated on the tip of a needle portion in a case of producing the microneedle array.

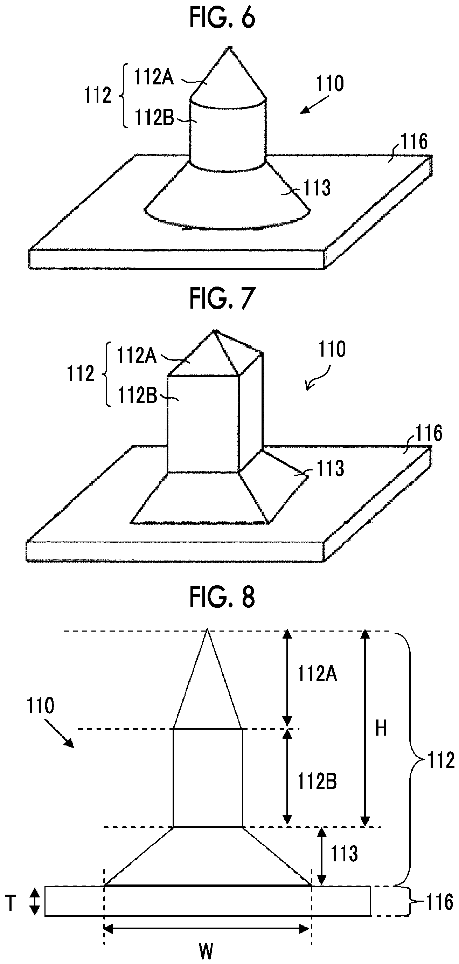

Figs. 6 and 7 illustrate microneedles 110 in different shapes.

The needle first layer 112A illustrated in Fig. 6 has a conical shape and the needle second layer 112B in Fig. 6 has a columnar shape. The needle first layer 112A illustrated in Fig. 7 has a square pyramid shape and the needle second layer 112B has a square columnar shape. However, the shape of the needle portion is not particularly limited.

Fig. 8 is a cross-sectional view illustrating the microneedles 110 illustrated in Figs. 6 and 7. In Fig. 8, H represents the height of the needle portion 112, W represents the diameter (width) of the base portion, and T represents the height (thickness) of the sheet portion 116.

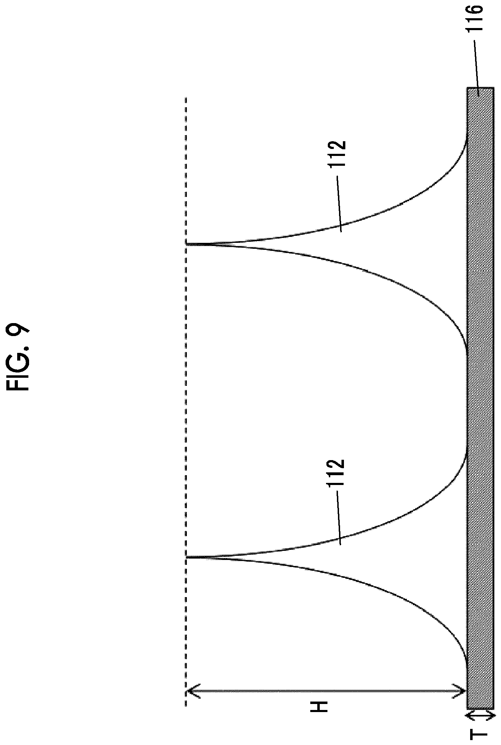

Fig. 9 is a cross-sectional view of a microneedle in another shape in which the inclination (angle) of the side surface of the needle portion 112 is continuously changed. In Fig. 9, H represents the height of the needle portion 112 and T represents the height (thickness) of the sheet portion 116.

In the microneedle array according to the embodiment of the present invention, it is preferable that needle portions are arranged at intervals of approximately 0.1 to 10 needles per 1 mm in a row. It is more preferable that the microneedle array has 1 to 10000 microneedles per 1 cm². In a case where the density of microneedles is set to 1 needle/cm² or greater, the microneedles can efficiently puncture the skin. Further, in a case where the density of the microneedles is set to 10000 needles/cm² or less, the microneedle array can sufficiently puncture the skin. The density of needle portions is preferably in a range of 10 to 5000 needles/cm², more preferably in a range of 25 to 1000 needles/cm², and particularly preferably in a range of 25 to 400 needles/cm².

The microneedle array according to the embodiment of the present invention can be supplied in a sealed storage form together with a drying agent. As the drying agent, known drying agents (such as silica gel, calcined lime, calcium chloride, silica alumina, and a sheet-like drying agent) can be used.

### [Method of producing microneedle array]

According to the present invention, there is provided a method of producing the microneedle array according to the embodiment of the present invention, including a step of concentrating influenza vaccine, a step of forming needle portions using the concentrated influenza vaccine obtained in the above-described step, and a step of forming a sheet portion.

It is preferable that the step of concentrating the influenza vaccine is a step of concentrating the influenza vaccine by centrifugation.

In the present invention, the needle portions can be formed by filling a mold with a liquid containing influenza vaccine, a water-soluble polymer, and meglumine or a salt thereof.

The microneedle array according to the embodiment of the present invention can be produced in conformity with the methods described in, for example, JP2013-153866A or WO2014/077242A.

### (Preparation of mold)

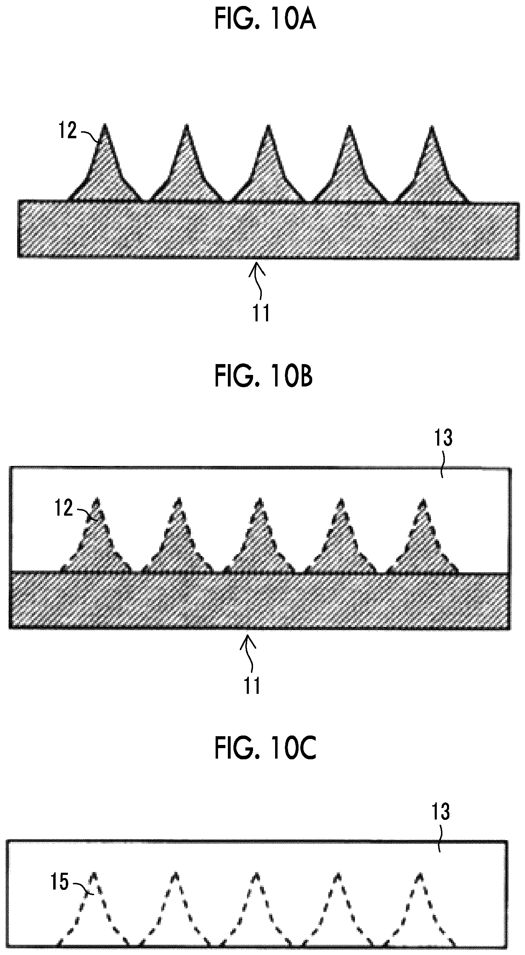

Figs. 10A to 10C are step views illustrating a method of preparing a mold (die). As illustrated in Fig. 10A, first, an original plate is prepared in order to prepare the mold. There are two methods for preparing an original plate 11.

According to the first method, a Si substrate is coated with a photoresist, exposed, and then developed. Further, an array of shaped portions 12 having a conical shape (projection) is prepared on the surface of the original plate 11 by performing etching using reactive ion etching (RIE) or the like. In a case where the etching such as RIE or the like is performed so as to form shaped portions having a conical shape on the surface of the original plate 11, the portions having a conical shape can be formed by performing etching in an oblique direction while the Si substrate rotates. According to the second method, an array of the shaped portions 12 having a square pyramid shape or the like is formed on the surface of the original plate 11 by performing processing on a metal substrate such as Ni using a cutting tool such as a diamond bit.

Next, a mold is prepared. Specifically, a mold 13 is prepared using the original plate 11 as illustrated in Fig. 10B. As the method of preparing the mold, four methods are considered.

According to the first method, a silicone resin obtained by adding a curing agent to polydimethylsiloxane (PDMS, for example, SYLGARD 184 (registered trademark, manufactured by Dow Corning Toray Co., Ltd.)) is poured into the original plate 11, subjected to a heat treatment at 100°C, cured, and peeled off from the original plate 11. According to the second method, an ultraviolet (UV) cured resin which is cured by being irradiated with ultraviolet rays is poured into the original plate 11, irradiated with ultraviolet rays in a nitrogen atmosphere, and peeled off from the original plate 11. According to the third method, a solution obtained by dissolving a plastic resin such as polystyrene or polymethyl methacrylate (PMMA) in an organic solvent is poured into the original plate 11 coated with a peeling agent, dried so that the organic solvent is volatilized, and cured, and then peeled off from the original plate 11. According to the fourth method, an inverted product is produced using Ni electroforming.

In this manner, the mold 13 formed by needle-like recesses 15, which have an inverted shape of the conical shape or the pyramid shape of the original plate 11, being two-dimensionally arranged is prepared. The mold 13 prepared in the above-described manner is illustrated in Fig. 10C.

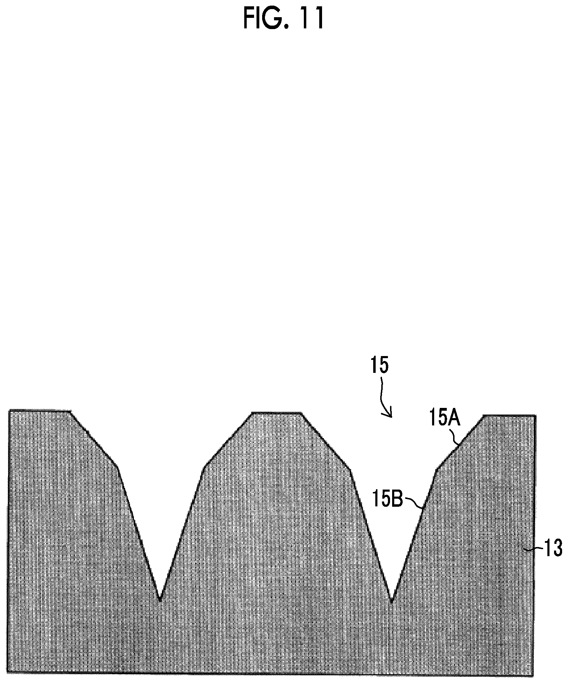

Fig. 11 illustrates another preferred embodiment of the mold 13. The needle-like recess 15 comprises a tapered inlet portion 15A which is narrower in a depth direction from the surface of the mold 13 and a tip recess 15B which is tapered in the depth direction. In a case where the inlet portion 15A has a tapered shape, the needle-like recess 15 is easily filled with the water-soluble polymer-dissolved solution.

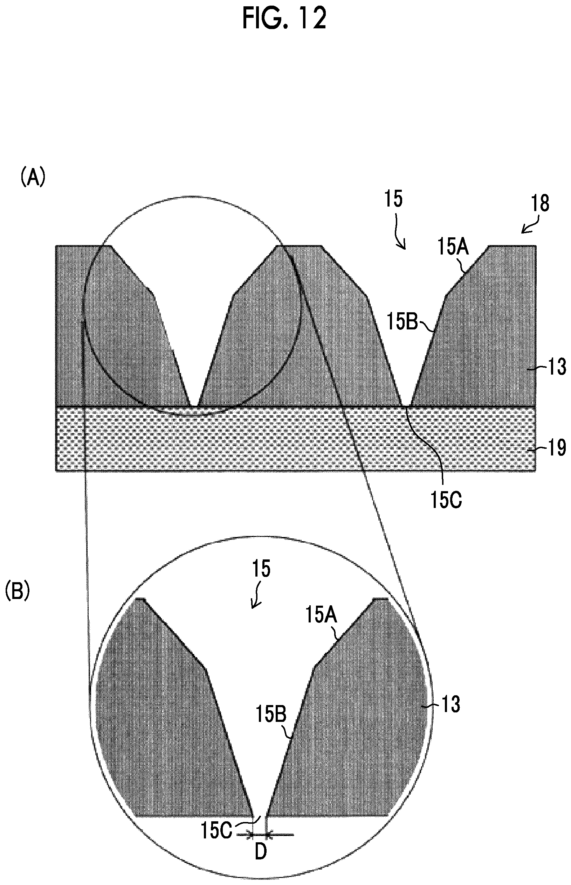

Fig. 12 illustrates a more preferred embodiment of a mold complex 18 in a case of producing the microneedle array. The (A) portion of Fig. 12 illustrates a mold complex 18. The (B) portion of Fig. 12 is a partially enlarged view of a portion enclosed by a circle in the (A) portion.

As illustrated in the (A) portion of Fig. 12, the mold complex 18 comprises the mold 13 having an air vent hole 15C formed on the tip (bottom) of the needle-like recess 15; and a gas permeating sheet 19 which is bonded to the rear surface of the mold 13 and is formed of a material that permeates a gas and does not permeate a liquid. The air vent hole 15C is formed as a through-hole penetrating the rear surface of the mold 13. Here, the rear surface of the mold 13 indicates the surface on a side where the air vent hole 15C is formed. With this configuration, the tip of the needle-like recess 15 communicates with the air through the air vent hole 15C and the gas permeating sheet 19.

In a case where such a mold complex 18 is used, only the air present in the needle-like recess 15 can be released from the needle-like recess 15 without permeation of the polymer-dissolved solution filling the needle-like recess 15. In this manner, the property of transferring the shape of the needle-like recess 15 to a polymer is excellent and a sharper needle portion can be formed.

A diameter D (diameter) of the air vent hole 15C is preferably in a range of 1 to 50 µm. In a case where the diameter D of the air vent hole 15C is less than 1 µm, the air vent hole 15C cannot be sufficiently used as an air vent hole. Further, in a case where the diameter D of the air vent hole 15C is greater than 50 µm, the sharpness of the tip of a formed microneedle is damaged.

As the gas permeating sheet 19 formed of a material that permeates a gas and does not permeate a liquid, for example, a gas permeating film (POREFLON (registered trademark), FP-010, manufactured by Sumitomo Electric Industries, Ltd.) can be suitably used.

As the material used for the mold 13, an elastic material or a metal material can be used. Among these, an elastic material is preferable and a material having a high gas permeability is more preferable. The oxygen permeability, which is a representative example of the gas permeability, is preferably 1×10⁻¹² (mL/s·m²·Pa) or greater and more preferably 1×10⁻¹⁰ (mL/s·m²·Pa) or greater. Further, 1 mL is 10⁻⁶ m³. In a case where the gas permeability is in the above-described range, the air present in a recess of the mold 13 can be released from the mold and a microneedle array with less defects can be produced. Specific examples of such materials include materials obtained by melting or dissolving, in a solvent, a silicone resin (for example, SYLGARD 184 (registered trademark, manufactured by Dow Corning Toray Co., Ltd.) or KE-1310ST (product number, manufactured by Shin-Etsu chemical Co., Ltd.)), a UV curable resin, or a plastic resin (for example, polystyrene or polymethyl methacrylate (PMMA)). Among these, a silicone rubber-based material is preferable since the material has durability to transfer resulting from repetitive pressure and has excellent peeling properties with respect to a material. Further, examples of the metal material include Ni, Cu, Cr, Mo, W, Ir, Tr, Fe, Co, MgO, Ti, Zr, Hf, V, Nb, Ta, a-aluminum oxide, zirconium oxide, stainless steel (for example, STAVAX (registered trademark) of Bohler-Uddeholm KK), and alloys thereof. As the material of a frame, the same material as the material of the mold 13 can be used.

### (Water-soluble polymer-dissolved solution)

In the present invention, it is preferable to prepare a water-soluble polymer-dissolved solution containing influenza vaccine and meglumine or a salt thereof used to form at least a part of a needle portion and a water-soluble polymer-dissolved solution used to form the sheet portion.

The type of water-soluble polymer is as described in the present specification above.

Disaccharides may be mixed into the water-soluble polymer-dissolved solution, and the kind of the disaccharides is as described in the present specification above.

Further, the solvent used for dissolution may be a solvent other than water as long as the solvent has volatility, and methyl ethyl ketone (MEK), an alcohol, or the like can be used as the solvent.

Specifically, the water-soluble polymer-dissolved solution containing a drug and meglumine or a salt thereof used to form at least a part of a needle portion is a liquid containing influenza vaccine, a water-soluble polymer, and meglumine or a salt thereof.

The concentration of the influenza vaccine in the liquid containing influenza vaccine, a water-soluble polymer, and meglumine or a salt thereof is not particularly limited, but is preferably in a range of 0.001 mg/mL to 100 mg/mL and more preferably in a range of 0.1 mg/mL to 20 mg/mL.

The concentration of the meglumine in the liquid containing influenza vaccine, a water-soluble polymer, and meglumine or a salt thereof is not particularly limited, but is preferably in a range of 0.001 mg/mL to 100 mg/mL and more preferably in a range of 0.1 mg/mL to 20 mg/mL.

The concentration of the water-soluble polymer in the liquid containing influenza vaccine, a water-soluble polymer, and meglumine or a salt thereof is not particularly limited, but is preferably in a range of 1 mg/mL to 100 mg/mL and more preferably in a range of 5 mg/mL to 50 mg/mL.

### (Formation of needle portion)

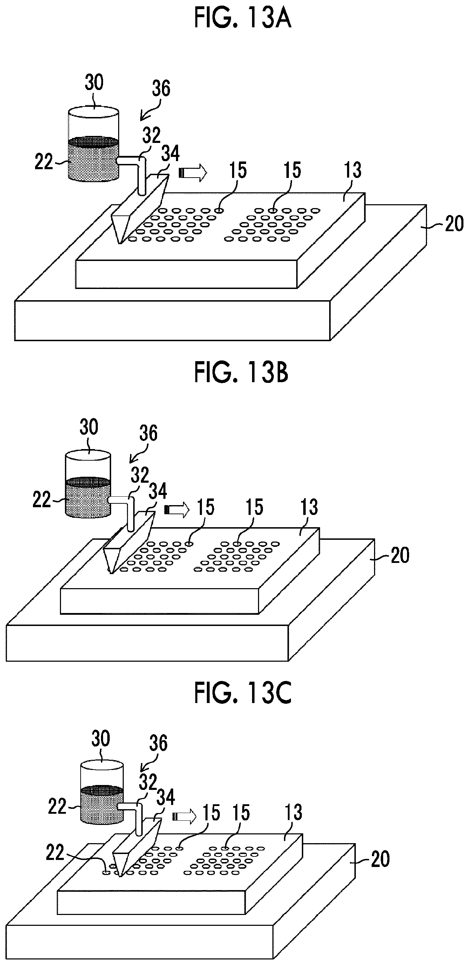

As illustrated in Fig. 13A, the mold 13 having needle-like recesses 15 which are two-dimensionally arranged is disposed on a base 20. In the mold 13, two sets of plural needle-like recesses 15 are formed such that 5 rows of needle-like recesses 15 and 5 columns of needle-like recesses 15 are two-dimensionally arranged. A liquid supply device 36 including a tank 30 which accommodates a water-soluble polymer-dissolved solution 22 containing a drug and meglumine or a salt thereof; a pipe 32 which is connected with the tank; and a nozzle 34 which is connected with the end of the pipe 32 is prepared. Further, in the present example, the case where 5 rows of needle-like recesses 15 and 5 columns of needle-like recesses 15 are two-dimensionally arranged is exemplified, but the number of the needle-like recesses 15 is not limited to 5 rows × 5 columns as long as the needle-like recesses are two-dimensionally arranged in a manner of M × N (M and N each independently represent an optional integer of 1 or greater, preferably in a range of 2 to 30, more preferably in a range of 3 to 25, and still more preferably in a range of 3 to 20).

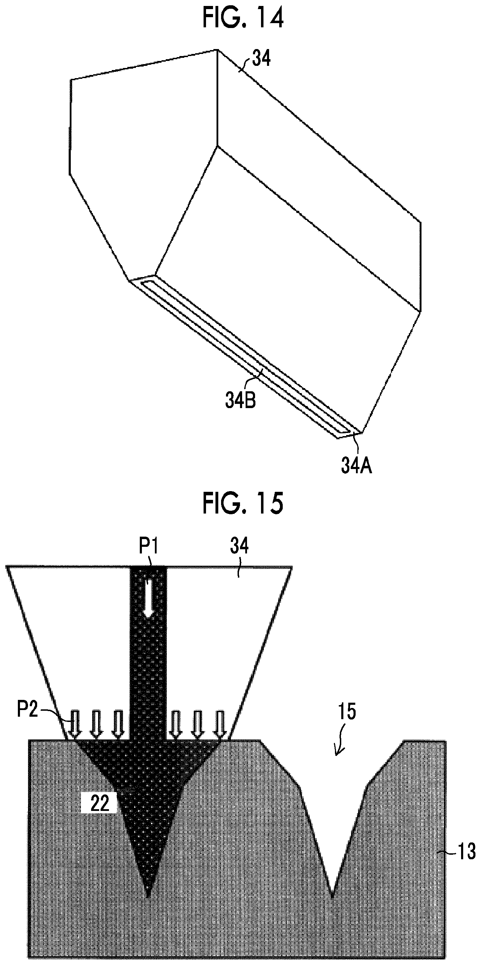

Fig. 14 is a perspective view schematically illustrating the end portion of the nozzle. As illustrated in Fig. 14, the end of the nozzle 34 comprises a lip portion 34A which is a flat surface and an opening portion 34B having a slit shape. For example, a plurality of needle-like recesses 15 forming one row can be concurrently filled with the water-soluble polymer-dissolved solution 22 containing a drug and meglumine or a salt thereof because of the opening portion 34B having a slit shape. The size (the length and the width) of the opening portion 34B is appropriately selected according to the number of needle-like recesses 15 to be concurrently filled with the solution. In a case where the length of the opening portion 34B is set to be large, a larger amount of needle-like recesses 15 can be filled with the polymer-dissolved solution 22 containing a drug at the same time. In this manner, the productivity can be improved.

As the material used for the nozzle 34, an elastic material or a metal material can be used. Examples thereof include TEFLON (registered trademark), stainless steel (steel special use stainless (SUS)), and titanium.

As illustrated in Fig. 13B, the position of the opening portion 34B of the nozzle 34 is adjusted on the needle-like recesses 15. The lip portion 34A of the nozzle 34 is in contact with the surface of the mold 13. The water-soluble polymer-dissolved solution 22 containing a drug and meglumine or a salt thereof is supplied to the mold 13 from the liquid supply device 36, and the needle-like recesses 15 are filled with the water-soluble polymer-dissolved solution 22 containing a drug and meglumine or a salt thereof from the opening portion 34B of the nozzle 34. In the present embodiment, the plurality of needle-like recesses 15 forming one row are concurrently filled with the water-soluble polymer-dissolved solution 22 containing a drug and meglumine or a salt thereof. However, the present invention is not limited thereto, and the needle-like recesses 15 can be filled with the solution one by one.

In a case where the mold 13 is formed of a material having a gas permeability, the water-soluble polymer-dissolved solution 22 containing a drug and meglumine or a salt thereof can be suctioned by suctioning the solution from the rear surface of the mold 13, and the filling of the needle-like recesses 15 with the water-soluble polymer-dissolved solution 22 containing a drug and meglumine or a salt thereof can be promoted.

Next to the filling step with reference to Fig. 13B, as illustrated in Fig. 13C, the liquid supply device 36 is relatively moved in the length direction and the vertical direction of the opening portion 34B while the lip portion 34A of the nozzle 34 is brought into contact with the surface of the mold 13, and the nozzle 34 is moved to the needle-like recesses 15 which have not been filled with the water-soluble polymer-dissolved solution 22 containing a drug and meglumine or a salt thereof. The position of the opening portion 34B of the nozzle 34 is adjusted on the needle-like recesses 15. In the present embodiment, the example of moving the nozzle 34 has been described, but the mold 13 may be moved.

Since the lip portion 34A of the nozzle 34 is brought into contact with the surface of the mold 13 and then the movement is made, the water-soluble polymer-dissolved solution 22 containing a drug and meglumine or a salt thereof, which remains on the surface of the mold 13 other than the needle-like recesses 15 can be collected by the nozzle 34. It is possible to prevent the water-soluble polymer-dissolved solution 22 containing a drug and meglumine or a salt thereof from remaining on the mold 13 other than the needle-like recesses 15.

In order to reduce the damage to the mold 13 and suppress deformation due to compression of the mold 13 as much as possible, it is preferable that the pressing pressure of the nozzle 34 against the mold 13 is set to be as small as possible during the movement. Further, in order to prevent the water-soluble polymer-dissolved solution 22 containing a drug and meglumine or a salt thereof from remaining on the surface of the mold 13 other than the needle-like recesses 15, it is preferable that at least one of the mold 13 or the nozzle 34 is formed of a flexible material which can be elastically deformed.

By repeating the filling step of Fig. 13B and the moving step of Fig. 13C, 5 rows and 5 columns of needle-like recesses 15 which are two-dimensionally arranged are filled with the water-soluble polymer-dissolved solution 22 containing a drug and meglumine or a salt thereof. In a case where 5 rows and 5 columns of needle-like recesses 15 which are two-dimensionally arranged are filled with the water-soluble polymer-dissolved solution 22 containing a drug and meglumine or a salt thereof, the liquid supply device 36 is moved to 5 rows and 5 columns of adjacent needle-like recesses 15 which are two-dimensionally arranged, and then the filling step of Fig. 13B and the moving step of Fig. 13C are repeated. The 5 rows and 5 columns of adjacent needle-like recesses 15 which are two-dimensionally arranged are also filled with the water-soluble polymer-dissolved solution 22 containing a drug and meglumine or a salt thereof.

The above-described filling step and moving step may be carried out in a mode (1) in which the needle-like recesses 15 are filled with the water-soluble polymer-dissolved solution 22 containing a drug and meglumine or a salt thereof while the nozzle 34 is moved or in a mode (2) in which the nozzle 34 is temporarily stopped on the needle-like recesses 15 during the movement of the nozzle 34, the needle-like recesses 15 are filled with the water-soluble polymer-dissolved solution 22 containing a drug and meglumine or a salt thereof, and the nozzle 34 is moved again after the filling. The lip portion 34A of the nozzle 34 is brought into contact with the surface of the mold 13 between the filling step and the moving step.

Fig. 15 is a partially enlarged view illustrating the end of the nozzle 34 and the mold 13 during the filling of the needle-like recesses 15 with the water-soluble polymer-dissolved solution 22 containing influenza vaccine and meglumine or a salt thereof. As illustrated in Fig. 15, the filling of the needle-like recesses 15 with the water-soluble polymer-dissolved solution 22 containing a drug and meglumine or a salt thereof can be promoted by applying a pressing pressure P1 into the nozzle 34. Further, in a case where the needle-like recesses 15 are filled with the water-soluble polymer-dissolved solution 22 containing a drug and meglumine or a salt thereof, it is preferable that a pressing force P2 for bringing the nozzle 34 into contact with the surface of the mold 13 is set to be greater than or equal to the pressing pressure P1 applied into the nozzle 34. In a case where the pressing force P2 is set to be greater than or equal to the pressing pressure P1, it is possible to suppress leakage of the water-soluble polymer-dissolved solution 22 containing a drug and meglumine or a salt thereof to the surface of the mold 13 from the needle-like recesses 15.

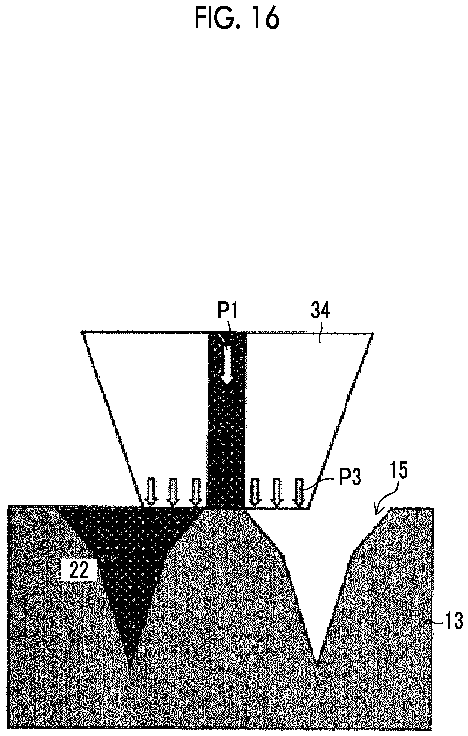

Fig. 16 is a partially enlarged view illustrating the end of the nozzle 34 and the mold 13 during the movement of the nozzle 34. In a case where the nozzle 34 is relatively moved with respect to the mold 13, it is preferable that a pressing force P3 of bringing the nozzle 34 into contact with the surface of the mold 13 is set to be smaller than the pressing force P2 of bringing the nozzle 34 into contact with the surface of the mold 13 during the filling. The pressing force P3 is set to be smaller than the pressing force P2 in order to reduce the damage to the mold 13 and suppress the deformation of the mold 13 due to compression.

In a case where the filling of the plurality of needle-like recesses 15 which are 5 rows and 5 columns of needle-like recesses is completed, the nozzle 34 is moved to the plurality of adjacent needle-like recesses 15 which are 5 rows and 5 columns of needle-like recesses. In regard to the liquid supply, in a case where the nozzle 34 is moved to the plurality of needle-like recesses 15 formed of 5 rows and 5 columns of adjacent needle-like recesses, it is preferable that the supply of the water-soluble polymer-dissolved solution 22 containing a drug and meglumine or a salt thereof is stopped. There is a distance between the needle-like recesses 15 in the fifth row and the needle-like recesses 15 in the next first row. In a case where the water-soluble polymer-dissolved solution 22 containing a drug and meglumine or a salt thereof is continuously supplied during the movement of the nozzle 34 between the rows, the liquid pressure inside of the nozzle 34 is extremely high in some cases. As the result, the water-soluble polymer-dissolved solution 22 containing a drug and meglumine or a salt thereof supplied from the nozzle 34 may flow out of the needle-like recesses 15 of the mold 13. In order to suppress the flowing out of the solution, it is preferable that the liquid pressure inside the nozzle 34 is detected and the supply of the water-soluble polymer-dissolved solution 22 containing a drug and meglumine or a salt thereof is stopped in a case where it is determined that the liquid pressure is extremely high.

Further, the method of supplying the water-soluble polymer-dissolved solution containing a drug and meglumine or a salt thereof using a dispenser that has a nozzle has been described, but bar coating, spin coating, spray coating, or the like can be applied in addition to the coating with the dispenser.

In the present invention, it is preferable that a drying treatment is performed after the water-soluble polymer-dissolved solution containing influenza vaccine and meglumine or a salt thereof to the needle-like recesses. That is, it is preferable that the method of producing the microneedle array according to the embodiment of the present invention includes a drying step of drying the solution after the filling step of filling the mold with the solution containing influenza vaccine, a water-soluble polymer, and meglumine or a salt thereof.

Further, it is preferable that the method of producing the microneedle array according to the embodiment of the present invention includes a step of coating the mold after the drying step with the water-soluble polymer-dissolved solution. That is, as one preferred example of the method of producing the microneedle array according to the embodiment of the present invention, a method including a step of drying a mold for forming needle portions filled with a first water-soluble polymer-dissolved solution containing influenza vaccine and meglumine or a salt thereof to form a part of the needle portions; and a step of filling the upper surfaces of the part of the needle portions which have been formed in the above-described manner with a second water-soluble polymer-dissolved solution and drying the mold can be exemplified.

It is preferable that the condition for drying the mold for forming needle portions filled with the first water-soluble polymer-dissolved solution containing influenza vaccine and meglumine or a salt thereof is set to be a condition that the water content of the first water-soluble polymer-dissolved solution reaches 20% or less after 30 to 300 minutes from the start of the drying of the mold.

It is particularly preferable that the drying can be controlled such that the temperature is held to be lower than or equal to a temperature at which the drug does not lose the effect and the water content of the water-soluble polymer-dissolved solution reaches 20% or less after 60 minutes or longer from the start of the drying of the mold.

As a method of controlling the drying rate, any method of delaying the drying, such as the temperature, the humidity, the drying air volume, the use of a container, and the volume and/or the shape of a container, can be employed.

It is preferable that the drying can be performed in a state where the mold for forming needle portions filled with the first water-soluble polymer-dissolved solution containing a drug is covered with a container or accommodated in a container.

The temperature during the drying is preferably in a range of 1°C to 45°C and more preferably in a range of 1°C to 40°C.

The relative humidity during the drying is preferably in a range of 10% to 95%, more preferably in a range of 20% to 95%, and still more preferably in a range of 30% to 95%.

### (Formation of sheet portion)

Several embodiments of a step of forming the sheet portion will be described.

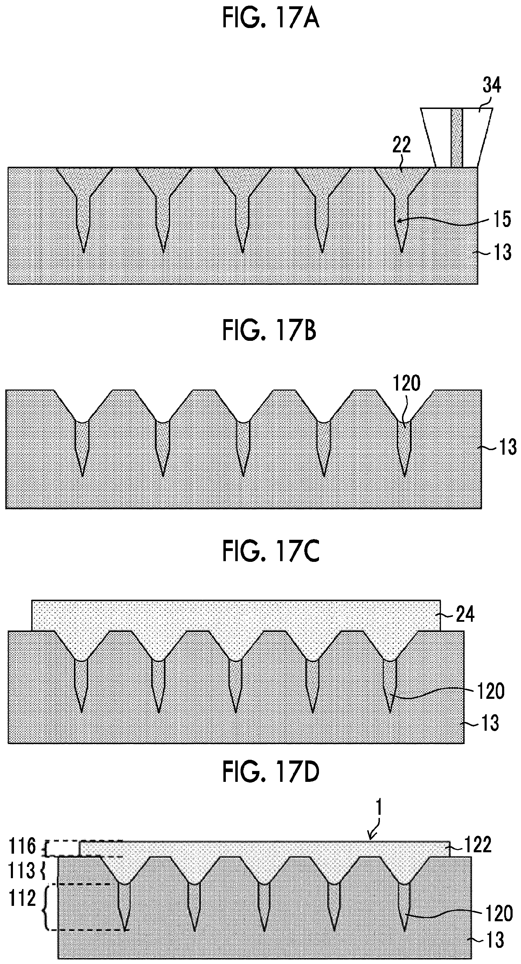

A first embodiment of a step of forming the sheet portion will be described with reference to Figs. 17A to 17D. The needle-like recesses 15 of the mold 13 are filled with the water-soluble polymer-dissolved solution 22 containing a drug and meglumine or a salt thereof from the nozzle 34. Next, as illustrated in Fig. 17B, a layer 120 containing a drug is formed in the needle-like recesses 15 by drying and solidifying the water-soluble polymer-dissolved solution 22 containing influenza vaccine and meglumine or a salt thereof. Subsequently, the mold 13 on which the layer 120 containing a drug has been formed is coated with a water-soluble polymer-dissolved solution 24 using a dispenser as illustrated in Fig. 17C. In addition to the coating using a dispenser, bar coating, spin coating, or spray coating can be applied. Since the layer 120 containing a drug is solidified, it is possible to suppress the diffusion of the drug in the water-soluble polymer-dissolved solution 24. Next, the microneedle array 1 formed of the plurality of needle portions 112, the frustum portions 113, and the sheet portion 116 is formed by drying and solidifying the water-soluble polymer-dissolved solution 24 as illustrated in Fig. 17D.

In the first embodiment, in order to promote the filling of the needle-like recesses 15 with the water-soluble polymer-dissolved solution 22 containing influenza vaccine and meglumine or a salt thereof and the water-soluble polymer-dissolved solution 24, it is preferable to apply a pressure from the surface of the mold 13 and perform suctioning from the rear surface of the mold 13 under reduced pressure.

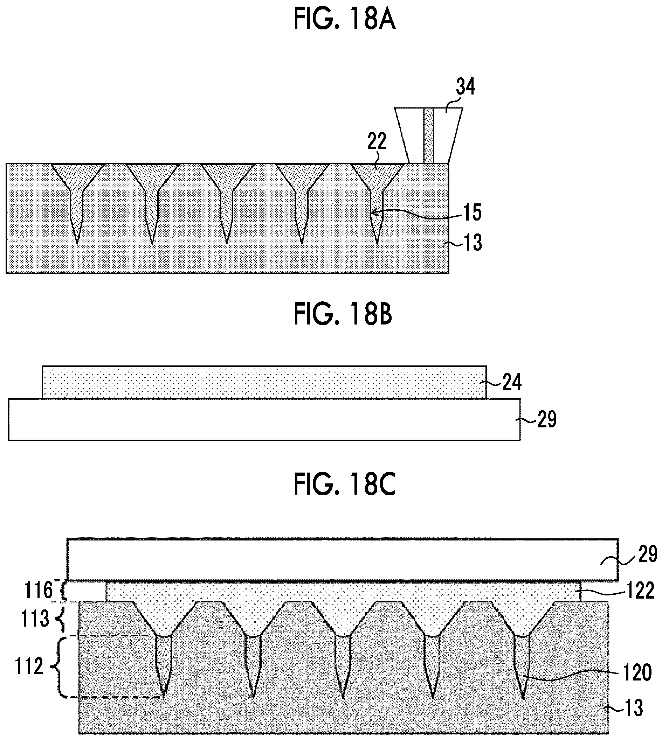

Next, a second embodiment will be described with reference to Figs. 18A to 18C. As illustrated in Fig. 18A, the needle-like recesses 15 of the mold 13 are filled with the water-soluble polymer-dissolved solution 22 containing a drug and meglumine or a salt thereof from the nozzle 34. Next, similarly to Fig. 17B, a layer 120 containing the drug is formed in the needle-like recesses 15 by drying and solidifying the water-soluble polymer-dissolved solution 22 containing a drug and meglumine or a salt thereof. Next, another support 29 is coated with the water-soluble polymer-dissolved solution 24 as illustrated in Fig. 18B. The support 29 is not limited, and examples of the support include polyethylene, polyethylene terephthalate, polycarbonate, polypropylene, an acrylic resin, triacetyl cellulose, and glass. Subsequently, the water-soluble polymer-dissolved solution 24 formed on the support 29 is superimposed on the mold 13 in which the layer 120 containing a drug has been formed on the needle-like recesses 15, as illustrated in Fig. 18C. In this manner, the needle-like recesses 15 are filled with the water-soluble polymer-dissolved solution 24. Since the layer 120 containing a drug is solidified, it is possible to suppress the diffusion of the drug in the water-soluble polymer-dissolved solution 24. Next, a microneedle array formed of the plurality of needle portions 112, the frustum portions 113, and the sheet portion 116 is formed by drying and solidifying the water-soluble polymer-dissolved solution 24.

In the second embodiment, in order to promote the filling of the needle-like recesses 15 with the water-soluble polymer-dissolved solution 24, it is also preferable to apply a pressure from the surface of the mold 13 and perform suctioning from the rear surface of the mold 13 under reduced pressure.

The method of drying the water-soluble polymer-dissolved solution 24 is not limited as long as the method includes a step of volatilizing the solvent in the polymer-dissolved solution. The method is not particularly limited, and a method of performing heating, blowing air, or decompression may be used. The drying treatment can be performed under the conditions of 1°C to 50°C for 1 to 72 hours. Examples of the method of blowing air include a method of blowing hot air at 0.1 to 10 m/sec. It is preferable that the drying temperature is a temperature at which the drug in the polymer-dissolved solution 22 containing a drug is not thermally degraded.

### (Peeling)

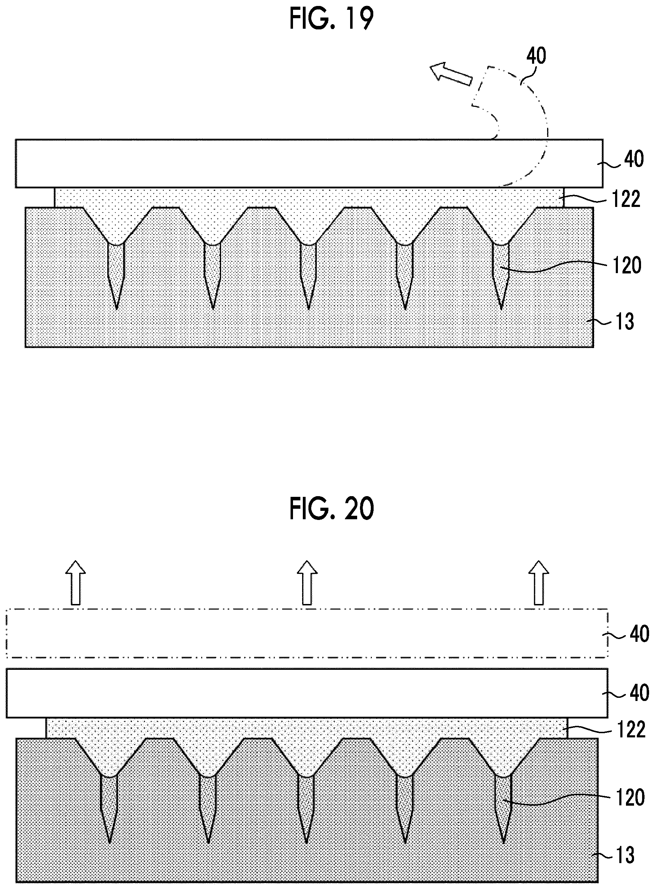

A method of peeling the microneedle array from the mold 13 is not particularly limited. It is preferable that needle portions are not bent or broken during the peeling. Specifically, a sheet-like base material 40 on which a pressure-sensitive adhesive layer is formed is attached onto the microneedle array and then the base material 40 can be peeled off from the end portion such that the base material 40 is turned over as illustrated in Fig. 19. However, the needle portions can be bent in a case of using this method. Therefore, as illustrated in Fig. 20, a method of disposing a sucking disc (not illustrated) on the base material 40 provided on the microneedle array and vertically pulling the base material up while suctioning the base material with air can be applied. Further, the support 29 may be used as the base material 40.

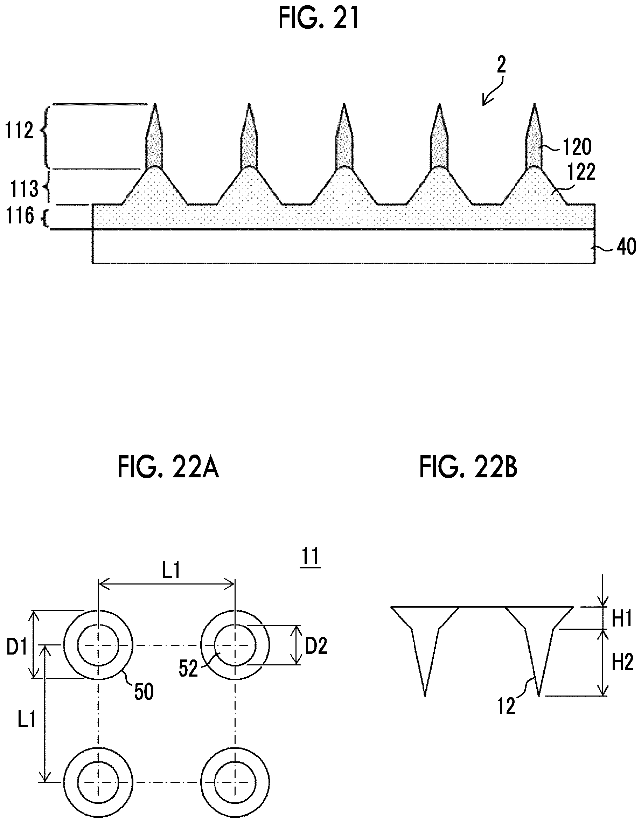

Fig. 21 illustrates a microneedle array 2 peeled off from the mold 13. The microneedle array 2 includes the base material 40, the needle portions 112 formed on the base material 40, the frustum portions 113, and the sheet portion 116. At least the tip of the needle portion 112 has a conical shape or a polygonal pyramid shape, but the shape of the needle portion 112 is not limited thereto.

The method of producing the microneedle array according to the embodiment of the present invention is not particularly limited, but it is preferable that the microneedle array is obtained by a production method including a step (1) of producing a mold, a step (2) of preparing a water-soluble polymer-dissolved solution containing influenza vaccine and meglumine or a salt thereof, a step (3) of filling the mold with the solution obtained in the step (2) to form upper end portions of the needle portions, a step (4) of filling the mold with a water-soluble polymer to form lower end portions of the needle portions and a sheet portion, and a step (5) of peeling the microneedle array from the mold.

Hereinafter, the present invention will be described in more detail with reference to examples of the present invention. Further, the materials, the use amounts, the ratios, the treatment contents, the treatment procedures, and the like shown in the following examples can be appropriately changed without departing from the spirit of the present invention. Therefore, the scope of the present invention should not be limitatively interpreted by the specific examples described below.

### Examples

### <Preparation of microneedle array containing influenza vaccine>

### (Production of mold)

An original plate 11 was prepared by arranging shaped portions 12 having a needle-like structure, on which a cone 52 with a diameter D2 of 340 µm and a height H2 of 834 µm was formed on a truncated cone 50 having a bottom surface with a diameter D1 of 800 µm and having a height H1 of 200 µm, on the surface of a smooth Ni plate having one side with a length of 40 mm, as illustrated in Fig. 22, and performing grinding processing on 100 needles having a pitch L1 of 1000 µm and a quadrangular shape in a two-dimensional square array. A film of silicon rubber (SILASTIC MDX 4-4210, manufactured by Dow Corning Toray Co., Ltd.) was formed on the original plate 11 such that the thickness thereof reached 0.6 mm, and the film was thermally cured in a state where 50 µm of the conical tip of the original plate 11 protruded from the film surface and then peeled off. In this manner, an inverted product of the silicon rubber having a through-hole with a diameter of approximately 30 µm was prepared. The silicon rubber inverted product which had 10 rows and 10 columns of needle-like recesses that were two-dimensionally arranged and formed on the central portion and in which the portion other than the plane portion in which each side had a length of 30 mm was cut off was used as a mold. A surface on which the opening portion of a needle-like recess was wide was set to the front surface of the mold and a surface having a through-hole (air vent hole) with a diameter of 30 µm was set to the rear surface of the mold.

### (Preparation of influenza vaccine concentrate)

The influenza vaccine stock solution was poured into a container for exclusive use for ultracentrifugation, and the influenza vaccine was allowed to be precipitated by ultracentrifugation (conditions: 131,491 × g, 90 minutes, 4°C). The supernatant was disposed of, phosphate buffered saline (PBS) was added thereto so that the mixture was vortexed, and the resultant was allowed to stand at 4°C overnight, thereby preparing an influenza vaccine concentrate.

### (Preparation of water-soluble polymer-dissolved solution containing influenza vaccine and meglumine)

An aqueous solution obtained by mixing the influenza vaccine concentrate, a water-soluble polymer, meglumine (FUJIFILM Wako Pure Chemical Corporation), saccharides, and Tween (registered trademark) 80 (MERCK) was prepared to obtain a water-soluble polymer-dissolved solution containing the influenza vaccine. As the water-soluble polymer, hydroxyethyl starch (HES) (Fresenius Kabi) and chondroitin sulfate (CS) (Maruha Nichiro Corporation) were used. As the saccharides, sucrose (Suc) (Japanese Pharmacopoeia grade, FUJIFILM Wako Pure Chemical Corporation), trehalose (Tre) (Hayashibara), and glucose (Japanese Pharmacopoeia grade, FUJIFILM Wako Pure Chemical Corporation) were used. The formulation of each solution is as listed in Tables 1, 2, and 3 below. The content of the influenza vaccine in Tables 1 and 3 was set to 9 mg per 1 mL. The content of the influenza vaccine in Table 2 was set to 0.3 mg per 1 mL.

### (Preparation of water-soluble polymer-dissolved solution forming sheet portion)

Chondroitin sulfate (Maruha Nichiro Corporation) was dissolved in water to prepare a water-soluble polymer-dissolved solution forming a sheet portion.

### (Filling and drying of polymer-dissolved solution containing drug)

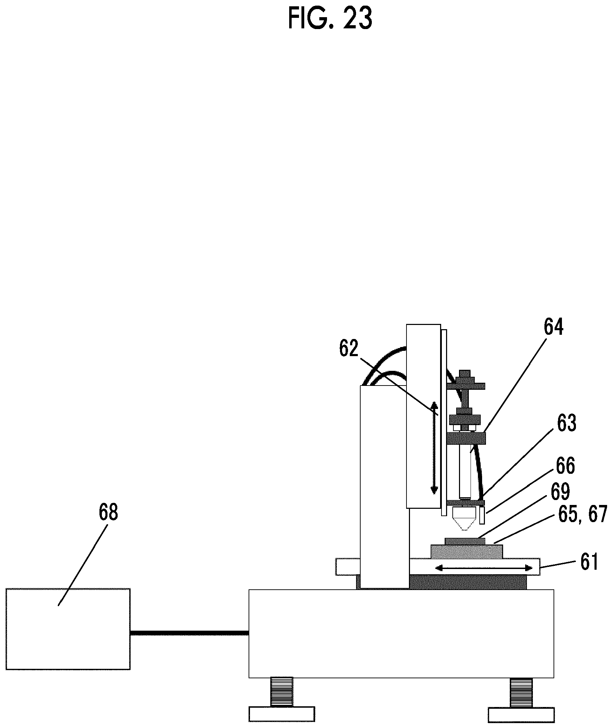

A filling device illustrated in Fig. 23 was used. The filling device comprises an X-axis drive unit 61 and a Z-axis drive unit 62 which control relative position coordinates of a mold and a nozzle; a liquid supply device 64 (ultratrace determination dispenser SMP-III, manufactured by Musashi Engineering, Inc.) to which the nozzle 63 is attachable; a suction stand 65 which fixes a mold 69; a laser displacement meter 66 (HL-C201A, manufactured by Panasonic Corporation) which measures the shape of the mold surface; a load cell 67 (LCX-A-500N, manufactured by Kyowa Electronic Instruments Co., Ltd.) which measures the pressing pressure of the nozzle; and a control mechanism 68 which controls the Z-axis based on data of measured values of the surface shape and the pressing pressure.

A gas-permeating film having one side with a length of 15 mm (POREFLON (registered trademark), FP-010, Sumitomo Electric Industries, Ltd.) was placed on a horizontal suction stand, and a mold was placed on the film such that the surface of the mold was directed to the upper side. The gas-permeating film and the mold were fixed to the vacuum stand by reducing the pressure with a suction pressure of a gauge pressure of 90 kPa in the rear surface direction of the mold.

A stainless steel (SUS) nozzle having a shape as illustrated in Fig. 14 was prepared, and a slit-like opening portion having a length of 12 mm and a width of 0.2 mm was formed at the center of a lip portion having a length of 20 mm and a width of 2 mm. The nozzle was connected to the liquid supply device. The liquid supply device and the nozzle were charged with a water-soluble polymer-dissolved solution containing 3 mL of a drug and meglumine or a salt thereof. The nozzle was adjusted such that the opening portion was parallel to the first row of a plurality of needle-like recesses formed on the surface of the mold. The nozzle was pressed against the mold with a pressure of 1.372×10⁴ Pa (0.14 kgf/cm²) at a position separated by 2 mm from the first row in the direction opposite to the second row. The nozzle was allowed to move 0.5 mm/min in a direction perpendicular to the length direction of the opening portion while the nozzle was pressed and the Z axis was controlled such that the fluctuation of the pressing pressure was in a range of ± 0.490×10⁴ Pa (0.05 kgf/cm²), the water-soluble polymer-dissolved solution containing a drug and meglumine or a salt thereof was released from the opening portion at 0.15 µL/sec for 20 seconds using the liquid supply device during the movement of the nozzle. The movement of the nozzle was stopped at a position separated by 2 mm after passing through the hole pattern of the plurality of needle-like recesses which had been two-dimensionally arranged, and the nozzle was separated from the mold.

The mold filled with the water-soluble polymer-dissolved solution containing influenza vaccine was allowed to stand in an environment of a temperature of 23°C and a relative humidity of 45% and dried.

### (Formation and drying of sheet portion)

The water-soluble polymer-dissolved solution forming the sheet portion was developed on the mold suctioning the solution in a state where the mold filled with the water-soluble polymer solution containing influenza vaccine was placed on a vacuum stand and sucked under reduced pressure. The suction was stopped after 60 minutes from the addition of the water-soluble polymer-dissolved solution, and the mold was allowed to stand in an environment of a temperature of 23°C and a relative humidity of 45% and dried.

### (Peeling)

The dried and solidified microneedle array was carefully peeled off from the mold to form a microneedle array containing influenza vaccine. Each microneedle is formed of a frustum portion and a needle portion. The height of a needle is approximately 800 µm and the width of a base portion is approximately 320 µm, and the frustum portion has a truncated cone structure such that the height thereof is approximately 160 µm, the diameter of the upper bottom surface thereof is approximately 320 µm, and the diameter of the lower bottom surface thereof is approximately 780 µm.The thickness of the sheet portion is approximately 200 µm, the number of needles is 100, the interval between needles is approximately 1 mm, and the needles are arranged in a square shape.

### <Evaluation of microneedle array>

### (Quantification of content of influenza vaccine in microneedle)

### (a) Content in microneedle from needle tip to 600 µm

Each needle portion of a microneedle having a needle length of 800 µm was cut at a position of 800 µm from the needle tip using a cutter blade. The cut needle portions were collected in a 1.5 mL tube. 0.5 mL of phosphate buffer was added to a 1.5 mL tube including the collected needle portions and stirred to dissolve the needle portions. The solution in which the needle portions were dissolved was diluted with phosphate buffer to have an appropriate concentration, and the content of the influenza vaccine contained in the cut needle portions was quantified according to an enzyme-linked immunosorbent assay (ELISA) method.

### (b) Content in microneedle from needle tip to 800 µm

Each remaining needle portion cut at 600 µm from the tip was cut at a position of the boundary between the needle portion and the frustum portion using a cutter blade. The remaining cut needle portions were collected in a 1.5 mL tube. 0.5 mL of phosphate buffer was added to a 1.5 mL tube including the collected needle portions and stirred to dissolve the needle portions. The solution in which the needle portions were dissolved was diluted with phosphate buffer to have an appropriate concentration, and the content of the influenza vaccine contained in the cut needle portions was quantified according to the ELISA method.

The total content obtained by adding the quantified content of influenza vaccine in the remaining needle portions to the content of influenza vaccine in the microneedles from the needle tips to 600 µm which had been quantified in (a) described above was set as the content of influenza vaccine in the microneedles from the needle tips to 800 µm.

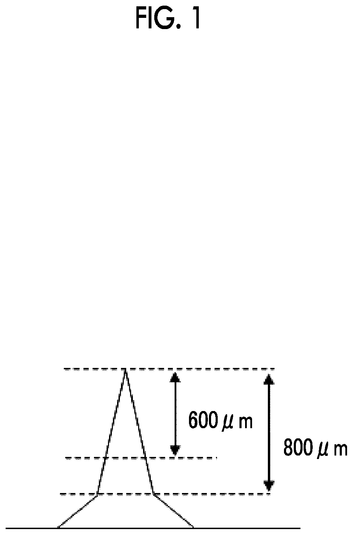

The region from the needle tip to 600 µm of a microneedle and the region from the needle tip to 800 µm of the microneedle are illustrated in Fig. 1. Further, the remaining needle portion in (b) described above corresponds to a region of 200 µm from the base portion in Fig. 1 (that is, a region obtained by excluding the region from the needle tip to 600 µm from the region from the needle tip to 800 µm).

In each microneedle array, the acquired results for the tip filling rate based on the following equation are listed in Tables 1, 2, and 3 below.

Tip filling rate = content of influenza vaccine in microneedle from needle tip to 600 µm/content of influenza vaccine in microneedle from needle tip to 800 µm

### (Stability of content of influenza vaccine in microneedle)

The produced influenza vaccine-including microneedle array was allowed to stand at 35°C for 3 days. After the standing, the microneedle array was removed, and the content of the vaccine was investigated. The content of the vaccine was evaluated according to the ELISA method.

The content of the vaccine after the standing at 35°C for 3 days to the content of the vaccine immediately after the production was set as the vaccine residual rate.

**[Table 1]**

| Formulation (Content ratio in case where content of influenza vaccine is set to 1) | | | Results | | Example/Comparative Example |
|---|---|---|---|---|---|
| Influenza vaccine | Hydroxyethyl starch | Meglumine | Influenza vaccine residual rate | Tip filling rate | |
| 1 | 3 | 0.010 | 60% | 68% | Example |
| 1 | 3 | 0.10 | 76% | 71% | Example |
| 1 | 3 | 1.0 | 80% | 80% | Example |
| 1 | 3 | 1.5 | 81% | 82% | Example |
| 1 | 3 | 3.0 | 81% | 83% | Example |
| 1 | 3 | 4.5 | 83% | 83% | Example |
| 1 | 3 | 7.6 | 85% | 84% | Example |
| 1 | 3 | 15 | 85% | 86% | Example |
| 1 | 3 | - | 40% | 59% | Comparative Example |

**[Table 2]**

| Formulation (Content ratio in case where content of influenza vaccine is set to 1) | | | Results | | Example/Comparative Example |
|---|---|---|---|---|---|
| Influenza vaccine | Hydroxyethyl starch | Meglumine | Influenza vaccine residual rate | Tip filling rate | |
| 1 | 68 | 0.03 | 72% | 72% | Example |
| 1 | 68 | 0.3 | 80% | 79% | Example |
| 1 | 68 | 3 | 84% | 83% | Example |
| 1 | 68 | 30 | 85% | 83% | Example |
| 1 | 68 | 90 | 85% | 84% | Example |
| 1 | 68 | 300 | 88% | 79% | Example |
| 1 | 68 | - | 42% | 65% | Comparative Example |

**[Table 3]**

| Formulation (Content ratio in case where content of influenza vaccine is set to 1) | | | | | | | | Results | | Example/Comparative Example |
|---|---|---|---|---|---|---|---|---|---|---|
| Influenza vaccine | Hydroxyethyl starch | Chondroitin sulfate | Meglumine | Sucrose | | Trehalos Glucose | Tween (registered rademark) 80 | Influenza vaccine residual rate | rip filling rate | |
| 1 | 1.5 | - | 1 | 1.5 | - | - | 0.1 | 88% | 95% | Example |
| 1 | 1.5 | - | 1 | - | 1.5 | - | 0.1 | 85% | 93% | Example |
| 1 | 1.5 | - | 1 | - | - - | 1.5 | 0.1 | 88% | 91% | Example |
| 1 | - | 1.5 | 1 | 1.5 | - | - | 0.1 | 83% | 95% | Example |
| 1 | - | 3.0 | 1 | - | - - | - | 0.1 | 78% | 83% | Example |

### Explanation of References

- 1:: microneedle array
- 2:: microneedle array
- 110:: microneedle
- 112:: needle portion
- 113:: frustum portion
- 116:: sheet portion
- 120:: layer containing polio vaccine
- 122:: layer that does not contain polio vaccine
- W:: diameter (width)
- H:: height
- T:: height (thickness)
- 11:: original plate
- 12:: shaped portion
- 13:: mold
- 15:: needle-like recess
- D:: diameter (diameter)
- 18:: mold complex
- 19:: gas permeating sheet
- 20:: base
- 22:: polio vaccine-containing solution
- 24:: water-soluble polymer-dissolved solution
- 29:: support
- 30:: tank
- 32:: pipe
- 34:: nozzle
- 34A:: lip portion
- 34B:: opening portion
- 36:: liquid supply device
- P1:: pressing pressure
- P2:: pressing force
- P3:: pressing force
- 40:: base material
- 50:: truncated cone
- 52:: cone
- D1:: diameter
- D2:: diameter
- L1:: pitch
- H1:: height
- H2:: height
- 61:: X-axis drive unit
- 62:: Z-axis drive unit
- 63:: nozzle
- 64:: liquid supply device
- 65:: suction stand
- 66:: laser displacement meter
- 67:: load cell
- 68:: control mechanism
- 69:: mold

## Claims

1. A self-dissolving microneedle array comprising:
a sheet portion; and
a plurality of needle portions which are present on an upper surface of the sheet portion,
wherein the needle portion contains a water-soluble polymer, influenza vaccine, and meglumine or a salt thereof, and the influenza vaccine is administered into a body by dissolution of the needle portions.

2. The microneedle array according to claim 1,
wherein a content of the water-soluble polymer contained in the needle portion is 10% by mass or greater and 99% by mass or less with respect to a solid content of the needle portion.

3. The microneedle array according to claim 1 or 2,
wherein a content of the meglumine or the salt thereof in the needle portion is in a range of 0.01 times to 300 times a content of the influenza vaccine.

4. The microneedle array according to any one of claims 1 to 3,
wherein the needle portion contains saccharides.

5. A method of producing the microneedle array according to any one of claims 1 to 4, the method comprising:
a step of concentrating influenza vaccine;
a step of forming needle portions using the concentrated influenza vaccine obtained in the above-described step; and
a step of forming a sheet portion.

6. The method of producing the microneedle array according to claim 5,
wherein the step of concentrating the influenza vaccine is a step of concentrating the influenza vaccine by centrifugation.
